(19) Europäisches Patentamt
European Patent Office
Office européen des brevets

(11) Numéro de publication : **0 128 833**
**B1**

(12) # FASCICULE DE BREVET EUROPÉEN

(45) Date de publication du fascicule du brevet : **25.03.87**

(51) Int. Cl.$^4$ : **B 03 D 1/00**, C 07 C153/07

(21) Numéro de dépôt : **84401180.9**

(22) Date de dépôt : **07.06.84**

(54) **Esters mono- et dithioiques, leurs préparation et applications.**

(30) Priorité : **10.06.83 FR 8309639**

(43) Date de publication de la demande :
**19.12.84 Bulletin 84/51**

(45) Mention de la délivrance du brevet :
**25.03.87 Bulletin 87/13**

(84) Etats contractants désignés :
**DE SE**

(56) Documents cités :
**GB-A- 1 282 493**

(73) Titulaire : **SOCIETE NATIONALE ELF AQUITAINE (PRODUCTION)**
**Tour Aquitaine**
**F-92400 Courbevoie (FR)**

(72) Inventeur : **Levesque, Guy**
**rue d'Ardennes Contest**
**F-14000 Caen (FR)**
Inventeur : **Tozzolino, Pierre**
**Chemin Rural Carrerot Serres-Morlaas**
**F-64160 Morlaas (FR)**

(74) Mandataire : **Kohn, Armand**
**5 Avenue Foch**
**F-92380 Garches (FR)**

**EP 0 128 833 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne l'application des esters mono- et di-thioïques dont le reste d'alcool porte au moins un groupe fonctionnel renfermant de l'oxygène à la flottation des minerais. L'invention comprend également des composés nouveaux ainsi qu'un procédé pour la préparation de ces composés.

Différents dérivés thiocarboxyliques sont connus et utilisés dans l'industrie, par exemple en cosmétique ou dans l'enrichissement de minerais. Tels sont par exemple les cas des dithioacides et de leurs esters, décrits dans FR-A-2 396 545, 2 504 131, 2 429 617 et 2 458 319. Toutefois, l'industrie ne s'est pas intéressée aux composés qui, à côté de leur fonction mono- ou bi-thioïque, portent d'autres groupes fonctionnels. Or, les travaux, à la base de la présente invention, ont montré justement que ce genre de composés ont une utilité accrue ; en effet, la modification de la solubilité ou de l'affinité pour différentes substances, notamment pour certains cations — qui résulte de la présence d'une seconde fonction dans la molécule du thio- ou di-thio-acide — conduit à des composés à activité améliorée. C'est le cas, par exemple, dans les utilisations des corps en question comme constituants de compositions cosmétiques, comme agents de flottation de minerais, complexants de métaux, etc.

Les produits utilisés dans l'application sont des thioesters ou des dithioesters dont le reste d'alcool comporte au moins un groupe hydroxyle ou carboxyle, libre ou combiné. Ce ou ces groupes additionnels peuvent se trouver en toute position possible par rapport à l'atome de O ou de S auquel est relié le reste d'alcool du thio- ou dithio-ester. La position la plus éloignée présente souvent des avantages.

Ces composés peuvent être représentés par la formule schématique, générale

$$R-\underset{\underset{S}{\|}}{C}-Y-R'-Z \qquad (1)$$

dans laquelle R est : un alkyle ou alkényle en $C_1$ à $C_{24}$, et plus particulièrement en $C_1$ à $C_{18}$, pouvant porter un phényle éventuellement alkylé ou un phényle, dont un ou plusieurs H peuvent être substitués par des alkyles ou des halogènes ;

Y est S ou O ;

R' est une chaîne aliphatique $-C_nH_{2n}-$ linéaire ou ramifiée, n étant 1 à 12, et, plus particulièrement, 1 à 6, des ramifications éventuelles étant de préférence en $-C_mH_{2m+1}$ avec m = 1 à 4 ;

Z représente une fonction —OH, —OR", —COOH, —COOR", $-CONH_2$, —CONHR" ou $-CON(R")_2$ où R" est un alkyle ou alkényle en $C_1$ à $C_8$, ou bien un phényle éventuellement alkylé, étant entendu que Z n'est pas obligatoirement au bout de la chaîne R', mais peut constituer une ramification de celle-ci.

Dans des composés particulièrement intéressants au point de vue pratique, R est un méthyle, éthyle ou propyle, ou bien un phényle, Y est S, R" est en $C_1$ à $C_6$, tandis que Z, étant —OH ou —COOH, se trouve en une position $\alpha$, $\beta$, $\gamma$ ou $\omega$ par rapport à Y.

Ainsi des exemples typiques des produits sont des dithio-acétates (éthane-dithioates), propane-dithioates, butane-dithioates, benzène-dithioates et toluène dithioates d'hydroxy- et de carboxy-méthyle, -éthyle, -propyle, iso-propyle, -butyle, -isobutyle, -tert.butyle, -pentyles, -hexyles, etc.

Les composés de la formule (1) dans laquelle Z est hydroxyle représentent des composés nouveaux.

L'invention comprend également un procédé de préparation des composés décrits ci-dessus, qui permet l'obtention de ces derniers avec bons rendements, à l'état de pureté convenable. Ce procédé utilise une réaction de Grignard, connue en soi, mais qui n'a pas été, jusqu'à présent, appliquée à des matières mises en œuvre suivant l'invention, ni dans les conditions pratiquées dans celle-ci.

Le procédé suivant l'invention consiste à employer un sel magnésien mixte d'un acide mono- ou di-thioïque,

$$(2) \qquad R-\underset{\underset{S}{\|}}{C}-O-MgX \qquad \text{ou} \qquad R-\underset{\underset{S}{\|}}{C}-S-MgX \qquad (2')$$

où R a la même signification que dans la formule (1) donnée plus haut, X étant un halogène ; on fait réagir ce sel avec un composé organique oxygéné, renfermant un atome actif, susceptible de se fixer sur le composé magnésien, après quoi le produit obtenu est traité par un acide pour éliminer le magnésium et son anion.

Des composés à atome actif, particulièrement utilisables, suivant l'invention, sont des aldéhydes, des époxydes et des sels d'acides organiques halogénés.

C'est pour la première fois, suivant l'invention, que l'on fait réagir un magnésien d'un groupe thioïque sur un aldéhyde, époxyde ou halogéno-acide, pour synthétiser un ester ou un acide mono- ou di-thioïque.

Ainsi, avec un aldéhyde, la première étape du procédé peut être représentée par l'équation :

$$R-\underset{\underset{S}{\|}}{C}-S-MgX + R''CHO \dashrightarrow R-\underset{\underset{S}{\|}}{C}-S-\underset{\underset{OMgX}{|}}{CHR''} \qquad (3)$$

Dans le cas d'un époxyde, la réaction peut s'écrire :

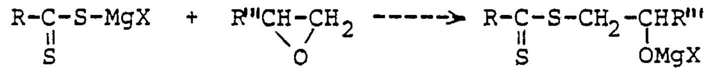

(4)

Lorsque le réactif employé est un sel d'halogénoacide organique du type X-$(CH_2)_n$COOM, où X est un halogène, n un entier, en général de 1 à 18, et M un cation, de préférence alcalin, le schéma suivant intervient :

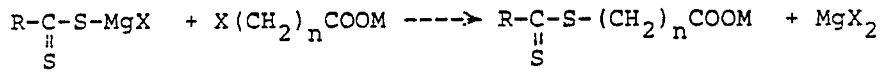

(5)

La seconde étape, qui consiste à traiter le magnésien complexe, obtenu par un acide tel que par exemple HCl, $SO_4H_2$, $ClO_4H$, etc. conduit à l'élimination de —MgX du produit formé par les réactions (3), (4) ou du cation M selon (5). Cela donne :

après la réaction (3) : 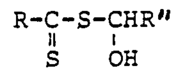

après la réaction (4) : 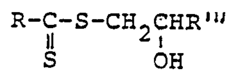

après la réaction (5) : 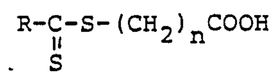

qui constituent les composés voulus selon l'invention:

La préparation d'un magnésien mixte, de départ, de formule (2) ou (2'), qui consiste à faire agir du sulfure de carbone $CS_2$ sur un magnésien RMgX dans un solvant, est connue depuis longtemps (J. Houben & L. Kesselkaul, Chem. Ber. 35, 1902, p. 3695). Il n'y a donc pas lieu de l'exposer ici.

Par contre, la première étape selon l'invention, dont l'objet est l'action d'un composé organique oxygéné, porteur d'un atome actif, sur ce magnésien mixte (2) ou (2'), bien qu'en quelque sorte analogue à des réactions d'halogénoalkyles avec de tels magnésiens (J. M. Beiner & A. Thuillier, C.R. Acad. Sc. Paris t.274-7.2.1972), elle présente des particularités nouvelles, ne découlant pas de la technique connue. On sait en effet que la réaction avec des halogénoalkyles devient d'autant plus difficile que la masse molaire de l'alkyle augmente ; il faut alors opérer à des températures d'environ 50 °C, ce qui affecte la pureté du produit obtenu (J. Meijer et col. Recueil de Travaux Chim. des Pays-Bas, 92, 1973, p. 602-4). Contrairement à cet art connu, dans le procédé de l'invention, les réactions (3) à (5), qui ont lieu au sein d'un solvant anhydre, sont conduites en général à des températures ne dépassant pas 20 °C, et de préférence entre 0° et 10 °C. Ainsi ne travaille-t-on ni aux basses températures de l'ordre de — 10° à — 15 °C prévues par certains auteurs, ni vers 50 °C, mais dans une marge spécifique de 0° à 20°, ou mieux entre 0° et 10 °C, qui permet une vitesse de réaction industriellement suffisante, tout en assurant une pureté convenable.

Le solvant, dans lequel ont lieu les réactions (3) à (5) peut être choisi parmi les différents liquides non réactifs vis-à-vis des composés magnésiens ; le tétra-hydrofuranne est particulièrement recommandable. Il présente également l'avantage de bien convenir à la préparation du magnésien de départ (2) ou (2') à partir d'un magnésien RMgX et du $CS_2$, on peut donc effectuer la réaction suivant l'invention dans le même milieu, dans lequel a eu lieu la préparation du magnésien (2) ou (2').

L'invention est illustrée non limitativement par les exemples qui suivent.

Exemples 1 à 5

On prépare d'abord 3 litres de solution, dans du tétrahydrofuranne, de 6 moles de sel magnésien mixte de l'acide dithioacétique

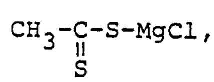

à la manière classique ; pour cela, 6 moles de $CH_3Cl$ sont mises à réagir avec 6 atomes de Mg dans

3 000 ml de tétrahydrofuranne, après quoi, on ajoute 6 moles de $CS_2$, le mélange étant maintenu à 5 °C, pendant une heure.

Suivant la caractéristique nouvelle de l'invention, à une fraction aliquote de 500 ml de solution obtenue, on ajoute 1 mole de réactif, qui varie d'un exemple à l'autre, et est précisé dans le tableau ci-dessous.

Le mélange ainsi formé est maintenu à 5 °C pendant 5 heures, pour laisser s'opérer la réaction (3) ou (4) indiquée plus haut.

On acidifie ensuite le milieu avec 200 ml d'HCl 7N, glacé, et l'on effectue une extraction des composés organiques présents au moyen du chlorure de méthylène ; l'extrait est lavé à HCl dilué, froid, puis neutralisé au bicarbonate de sodium. Après un lavage final à l'eau, l'extrait organique est séché sur du $Na_2SO_4$ anhydre. Une fois les solvants évaporés, le produit restant est distillé sous pression réduite.

Voici les désignations des réactifs utilisés et les caractéristiques des composés obtenus.

| Ex. | Réactif | Composé obtenu | Sa tempér. d'ébullition sous mm Hg : |
|---|---|---|---|
| 1 | Formaldéhyde | Dithioacétate d'hydroxyméthyle (Rendement 85%) $CH_3CS_2CH_2OH$ | 54-56°/13 mm |
| 2 | Acétaldéhyde | Dithioacétate d'hydroxy-1 éthyle (Rendement 90%) $CH_3CS_2\underset{OH}{CH}-CH_3$ | 31-33°/1 mm |
| 3 | Oxyde d'éthylène | Dithioacétate d'hydroxy-2 éthyle (Rendement 80%) $CH_3CS_2CH_2CH_2OH$ | 78-80°/13 mm |
| 4 | Epoxy-1,2 butane | Dithioacétate d'hydroxy-2 butyle (Rendement 73 %) $CH_3CS_2CH_2\underset{OH}{CH_2}C_2H_5$ | 74-76°/13 mm |
| 5 | Epoxy-1,2 isobutane | Dithioacétate d'hydroxy-2 méthyl-2 propyle (Rendement 92%) $CH_3CS_2CH_2-\underset{OH}{C}(CH_3)_2$ | 75-77/13 mm |

Exemple 6

La préparation du magnésien est analogue à celle des exemples précédents, mais le chlorure de méthyle $CH_3Cl$ est remplacé par celui de phényle, $C_6H_5Cl$, ce qui conduit à une solution de sel magnésien mixte de l'acide benzène-dithioïque

$$C_6H_5-\underset{\underset{S}{\parallel}}{C}-S-MgCl.$$

Celle-ci est traitée avec du formaldéhyde pris à l'état gazeux, à raison de 1 mol. pour 1 mol. de magnésien. Le produit obtenu, avec un rendement de 72 %, est le benzène-dithioate d'hydroxyméthyle, $C_6H_5—CS_2—CH_2OH$, bouillant à 82-86° sous 0,1 mm Hg.

Les exemples 1 à 6, comparés à ceux du Tableau II, page 644 des Comptes Rendus de l'Acad. Sc. Paris t. 274, 7.2.1972, signalé plus haut, montrent une forte amélioration des rendements, 72 à 92 % contre 37 à 71 %, par rapport à la fixation d'alkyles ou du phényle ne portant aucun groupe fonctionnel.

Exemples 7 à 11

Réaction de sels magnésiens mixtes d'acides dithioïques avec du chloro-acétate de sodium

Dans chaque exemple, on part d'un autre composé

$$R-\underset{\underset{S}{\parallel}}{C}-S-MgCl$$

préparé à partir d'un RCl différent, selon le mode opératoire des exemples 1 à 5.

A chacune des solutions obtenues, on ajoute 1 mole de $Cl\text{-}CH_2COONa$ en solution concentrée, à 43 % dans l'eau, par mole de dithioate magnésien.

Après 24 heures, à 20 °C, la solution résultante est concentrée par évaporation sous pression réduite, puis acidifiée avec 200 ml d'HCl 12N. La phase organique est décantée. Par purification chromatographique sur silice, on a obtenu les dithioesters de carboxyméthyle suivants.

| Exemple n° | R du magnésien de départ | Rendement % | Ester obtenu | Son point de fusion |
|---|---|---|---|---|
| 7 | 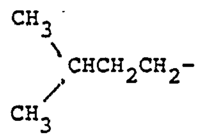 | 63 | 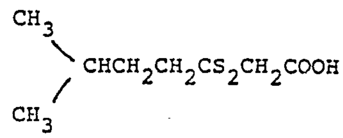 | 35°C |
| 8 | $CH_3$- | 47 | $CH_3CS_2CH_2COOH$ | 77°C |
| 9 | $CH_3CH_2CH_2CH_2$- | 66 | $CH_3(CH_2)_3CS_2CH_2COOH$ | 13°C |
| 10 | $CH_3(CH_2)_6$- | 61 | $CH_3(CH_2)_6CS_2CH_2COOH$ | 49°-50°C |
| 11 | $C_6H_5$- | 65 | $C_6H_5\text{-}CS_2CH_2COOH$ | 122°C |

## Applications

Les exemples 12 à 22, qui suivent, illustrent l'application de l'invention à quelques minéraux particuliers. Le mode opératoire, appliqué dans ces exemples, comportait le traitement d'une pulpe constituée par 1 g de minéral en particules de 63 à 160 microns, dans 300 ml d'eau, cette pulpe étant placée dans une cellule de Hallimond.

Sous agitation magnétique, on ajoute de l'acide sulfurique ou de la soude, de façon à ajuster le pH de la pulpe à la valeur voulue. Après l'addition d'une quantité appropriée de composé thioïque, en solution dans l'alcool éthylique ou dans du tétrahydrofuranne, à la pulpe, on fait passer un courant d'azote d'environ 10 l/h à la base de la cellule, à travers un filtre fritté n° 3. L'opération de flottation proprement dite est effectuée durant 3 minutes. Les particules de minéral, entraînées à la surface, sont récupérées, séchées et pesées ; on détermine ainsi le pourcent de la quantité récupérée, flottée, de ce minéral par rapport à la pulpe traitée.

Les essais ont été effectués avec 0,1 ml d'une solution alcoolique ou THF à 1/1 000 de collecteur, ce qui correspond à 100 g de collecteur par tonne de minéral.

Pour comparaison, les exemples 20-22 portent sur l'amyl xanthate de K connu comme le meilleur des collecteurs employés industriellement jusqu'à présent.

Tous les essais ont été effectués à la température ambiante.

Le tableau, à la page suivante, donne les résultats de ces essais.

(Voir Tableau page 6)

Exemples 12 à 22

| Ex n° | Composé | pH | % flotté : galène | Blende | Chalco pyrite | Pyrite |
|---|---|---|---|---|---|---|
| 12- | $CH_3(CH_2)_3CSCH_2OH$ (‖ S)<br>Dithiopentanoate d'hydroxy-méthyle | 5,5 | 93 | 92,5 | 78 | 77 |
| 13- | " " " | 7,05 | 90 | 90 | 70,5 | 70 |
| 14- | " " " | 9,02 | 96 | 15 | 87 | 80,5 |
| 15- | " " " | 10,50 | 93 | 16 | 84 | 86 |
| 16- | $CH_3-CH(CH_3)CH_2CH_2CSCH_2OH$ (‖ S)<br>Dithio méthyl-4 pentanoate d'hydroxy-méthyle | 5,55 | 92 | 95 | 68 | 52 |
| 17- | " " " | 7,07 | 92 | 93,5 | 66 | 31 |
| 18- | " " " | 9,01 | 94 | 39 | 80 | 51 |
| 19- | " " " | 10,50 | 92 | 17 | 79 | 76 |
| | Amylxanthate de potassium | 5,5 | 90 | | | |
| 20- | " " | 7,05 | 86 | | | |
| 21- | " " | 9,00 | 85 | | | |
| 22- | " " | 10,50 | 79 | | | |

Les résultats obtenus prouvent tout l'intérêt des composés suivant l'invention pour la flottation de minerais. Dans le cas de la galène, les meilleurs rendements sont atteints avec des esters dans l'acide dithioïque est en $C_5$ à $C_8$ (exemples 12-19) et, fait remarquable, on peut opérer avantageusement à tous pH de 5 à 11. Ces esters sont supérieurs à l'excellent collecteur de la technique connue, l'amylxanthate de potassium, dont l'efficacité baisse dès le pH 7 (exemples 20 à 22).

A la blende conviennent encore très bien ces mêmes esters des acides dithioïques, à des pH ne dépassant pas 7 ; vers le pH 10 le taux de minéral flotté devient faible (surtout exemples 14, 15 et 19), ce dont on peut tirer avantage pour des séparations d'avec la galène ou/et la chalcopyrite.

**Revendications**

1. Application des esters mono- ou di-thioïques à la flottation de minerais, en tant que collecteurs, caractérisée en ce que les esters utilisés répondent à la formule

$$R-\underset{\underset{S}{\|}}{C}-Y-R'Z$$

dans laquelle R est : un alkyle ou alkényle en $C_1$ à $C_{24}$, et plus particulièrement en $C_1$ à $C_{18}$, pouvant porter un phényle éventuellement alkylé ou un phényle dont un ou plusieurs H peuvent être substitués par des alkyles ou des halogènes ;

Y est S ou O ;

R' est une chaîne aliphatique $—C_nH_{2n}—$ linéaire ou ramifiée, n étant 1 à 12, et, plus particulièrement, 1 à 6, des ramifications éventuelles étant de préférence en $—C_mH_{2m+1}$ avec m = 1 à 4 ;

Z représente une fonction —OH, —OR", —COOH, —COOR", $—CONH_2$, —CONHR" ou $—CON(R")_2$ où R" est un alkyle ou alkényle en $C_1$ à $C_8$, ou bien un phényle éventuellement alkylé, étant entendu que Z n'est pas obligatoirement au bout de la chaîne R', mais peut constituer une ramification de celle-ci.

2. Application suivant la revendication 1, caractérisée en ce que R est un méthyle, éthyle ou propyle, ou bien un phényle, Y est S, R" est en $C_1$ à $C_6$, tandis que Z, étant —OH ou —COOH, se trouve en une position α, β, γ ou ω par rapport à Y.

3. Application suivant la revendication 1, caractérisée en ce que le collecteur employé est le dithiopentanoate ou le dithiométhyle-4-pentanoate d'hydroxyméthyle, en particulier dans la flottation des galènes, blende, chalcopyrite ou/et pyrite.

4. Nouveau produit chimique, constitué par un ester mono- ou di-thioïque de formule

$$R-\underset{\underset{S}{\|}}{C}-Y-R'-OH$$

dans laquelle R est : un alkyle ou alkényle en $C_1$ à $C_{24}$, et plus particulièrement en $C_1$ à $C_{18}$, pouvant porter un phényle éventuellement alkylé ou un phényle dont un ou plusieurs H peuvent être substitués par des alkyles ou des halogènes ;

Y — est S ou O ;

R' — est une chaîne aliphatique $—C_nH_{2n}—$ linéaire ou ramifiée, n étant 1 à 12, et, plus particulièrement, 1 à 6, des ramifications éventuelles étant de préférence en $—C_mH_{2m+1}$ avec m = 1 à 4 ;

le groupe —OH pouvant se trouver en tout point de la chaîne R'.

5. Produit suivant la revendication 4, caractérisé en ce que R' est une chaîne aliphatique.

6. Produit suivant la revendication 4, constitué par du dithioacétate d'hydroxy-méthyle, d'hydroxy-1 éthyle, d'hydroxy-2 éthyle, d'hydroxy-2-butyle ou d'hydroxy-2 méthyl-2 propyle.

7. Produit suivant la revendication 4, constitué par du dithiopentanoate, dithiohexanoate, dithiooctanoate ou dithiobenzoate d'hydroxy-méthyle.

8. Procédé de production d'esters mono- et di-thioïques suivant la revendication 4, caractérisé en ce que l'on fait réagir un aldéhyde ou un époxyde, avec un sel magnésien mixte d'un acide mono- ou dithioïque

$$R-\underset{\underset{S}{\|}}{C}-Y-MgX$$

où X est un halogène, et que le produit, ainsi obtenu, est traité par un acide aqueux.

9. Procédé suivant la revendication 8, caractérisé en ce que l'aldéhyde est le formaldéhyde ou l'acétaldéhyde, et l'époxyde est l'époxy-éthane, époxy-propane, époxy-butane ou époxy-isobutane.

10. Procédé suivant la revendication 8 ou 9, dans lequel les réactifs sont mis en contact au sein d'un solvant, caractérisé en ce que la préparation est conduite à une température de 0° à 20 °C.

**Claims**

1. The use of mono- or di-thioic esters for the flotation of minerals, as collectors, characterised in that the esters utilised correspond to the formula

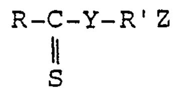

in which R is a $C_1$ to $C_{24}$ and more particularly a $C_1$ to $C_{18}$ alkyl or alkenyl group, which can carry a phenyl group which may be alkylated, or a phenyl group where one or more H atoms can be substituted by alkyl groups or halogen atoms ;

Y is S or O ;

R' is a straight or branched $—C_nH_{2n}—$ aliphatic chain, n being 1 to 12 and more particularly 1 to 6, any side chains preferably being $—C_mH_{2m+1}$ with m = 1 to 4 ;

Z represents a —OH, —OR", —COOH, —COOR", $—CONH_2$, —CONHR" or $—CON(R")_2$ function, where R" is a $C_1$ to $C_8$ alkyl or alkenyl group or a phenyl group which may be alkylated, it being understood that Z is not necessarily at the end of the R' chain, but can constitute a side chain thereof.

2. The use according to claim 1, characterised in that R is a methyl, ethyl or propyl group or a phenyl group, y is S, R" is $C_1$ to $C_6$, while Z is —OH or —COOH, located in an α, β, γ, or ω position with respect to Y.

3. The use according to claim 1, characterised in that the collector employed is hydroxymethyl dithiopentanoate or 4-dithiomethylpentanoate, in particular in the flotation of galenas, blende, chalcopyrite and/or pyrites.

4. A new chemical product, comprising a mono- or dithionic ester of the formula :

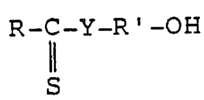

in which R is a $C_1$ to $C_{24}$ and more particularly a $C_1$ to $C_{18}$ alkyl or alkenyl group, which can carry a phenyl group which may be alkylated or a phenyl group where one or more of the H atoms can be substituted by alkyl groups or halogen atoms ;

Y is S or O ;

R' is a straight or branched $—C_nH_{2n}—$ aliphatic chain, n being 1 to 12 and more particularly 1 to 6, any side chains preferably being $—C_mH_{2m+1}—$ with m = 1 to 4 ;

the group —OH possibly being located at the end of the R' chain.

5. A product according to claim 4, characterised in that R' is an aliphatic chain.

6. A product according to claim 4, comprising hydroxymethyl, 1-hydroxyethyl, 2-hydroxyethyl, 2-hydroxybutyl or 2-hydroxy-2-methylpropyl dithioacetate.

7. A product according to claim 4, comprising hydroxymethyl, dithiopentanoate, dithiohexanoate, dithiooctanoate or dithiobenzoate.

8. A process of production of mono- and dithioic esters according to claim 4, characterised in that an aldehyde or an epoxide is reactive with a mixed magnesium salt of a mono- or dithioic acid

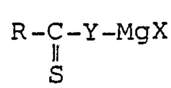

where X is a halogen atom and that the product so obtained is treated with an aqueous acid.

9. A process according to claim 8, characterised in that the aldehyde is formaldehyde or acetaldehyde and the epoxide is epoxyethane, epoxypropane, epoxybutane or expoxyisobutane.

10. A process according to claim 8 or 9, in which the reactants are contacted in a solvent, characterised in that the preparation is carried out at a temperature of 0° to 20 °C.

**Patentansprüche**

1. Verwendung von Mono- oder Dithiosäureestern als Kollektoren bei der Flotation von Erzen, dadurch gekennzeichnet, dass die verwendeten Ester der Formel

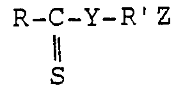

entsprechen, worin R ein Alkyl oder Alkenyl mit 1 bis 24 C-Atomen und insbesondere mit 1 bis 18 C-Atomen ist, die ein gegebenenfalls alkyliertes Phenyl oder ein Phenyl, von dem eines oder mehrere H-Atome durch Alkylreste oder Halogenatome ersetzt sein können, tragen können ;

Y S oder O ist ;

R′ eine lineare oder verzweigte aliphatische Kette $—C_nH_{2n}—$ ist, worin n 1 bis 12 und insbesondere 1 bis 6 ist, wobei die gegebenenfalls vorhandenen Verzweigungen vorzugsweise der Formel $—C_mH_{2m+1}—$ mit m = 1 bis 4 entsprechen ;

Z eine der funktionellen Gruppen —OH, —OR″, —COOH, —COOR″, $—CONH_2$, —CONHR″ oder $—CON(R″)_2$ ist, worin R″ ein Alkyl oder Alkenyl mit 1 bis 8 C-Atomen oder ein gegebenenfalls alkyliertes Phenyl ist, wobei Z sich nicht notwendigerweise am Ende der Kette R′ befindet, sondern eine Verzweigung derselben darstellen kann.

2. Verwendung nach Anspruch 1, dadurch gekennzeichnet, dass R Methyl, Äthyl oder Propyl oder auch Phenyl ist, Y S ist, R″ 1 bis 6 Kohlenstoffatome aufweist, während Z, das —OH oder —COOH ist, sich in α-, β-, γ- oder ω-Stellung, bezogen auf Y, befindet.

3. Verwendung nach Anspruch 1, dadurch gekennzeichnet, dass der verwendete Kollektor das Hydroxymethyl-dithiopentanoat oder das Hydroxymethyl-dithiomethyl-4-pentanoat, insbesondere bei der Flotation von Galenit, Blende, Chalkopyrit und/oder Pyrit, ist.

4. Neues chemisches Produkt, bestehend aus einem Mono- oder Dithiosäureester der Formel

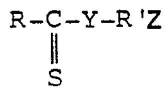

worin R ein Alkyl oder Alkenyl mit 1 bis 24 C-Atomen und insbesondere mit 1 bis 18 C-Atomen ist, die ein gegebenenfalls alkyliertes Phenyl oder ein Phenyl, von dem eines oder mehrere H-Atome durch Alkylreste oder Halogenatome substituiert sind, tragen können ;

Y S oder O ist ;

R′ eine lineare oder verzweigte aliphatische Kette $—C_nH_{2n}—$ ist, n 1 bis 12 und insbesondere 1 bis 6 ist, wobei die gegebenenfalls vorhandenen Verzweigungen vorzugsweise der Formel $—C_mH_{2m+1}$ mit m = 1 bis 4 entsprechen ;

wobei die Gruppe —OH sich an sämtlichen Stellen der Kette R′ befinden kann.

5. Produkt nach Anspruch 4, dadurch gekennzeichnet, dass R′ eine aliphatische Kette ist.

6. Produkt nach Anspruch 4, bestehend aus Hydroxymethyl-, Hydroxy-1-ethyl-, Hydroxy-2-ethyl-, Hydroxy-2-butyl- oder Hydroxy-2-methyl-2-propyl-dithioacetat.

7. Produkt nach Anspruch 4, bestehend aus Hydroxymethyldithiopentanoat, -dithiohexanoat, -dithiooctanoat oder -dithiobenzoat.

8. Verfahren zur Herstellung der Mono- und Dithiosäureester nach Anspruch 4, dadurch gekennzeichnet, dass man einen Aldehyd oder ein Epoxid mit einem gemischten Magnesiumsalz einer Mono- oder Dithiosäure

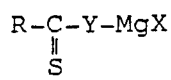

worin X ein Halogen bedeutet, umsetzt und das auf diese Weise erhaltene Produkt mit einer wässerigen Säure behandelt.

9. Verfahren nach Anspruch 8, dadurch gekennzeichnet, dass der Aldehyd, Formaldehyd oder Acetaldehyd ist und das Epoxid Epoxyethan, Epoxypropan, Epoxybutan oder Epoxyisobutan ist.

10. Verfahren nach Anspruch 8 oder 9, bei dem die Reaktionsteilnehmer in einem Lösungsmittel in Kontakt miteinander gebracht werden, dadurch gekennzeichnet, dass die Herstellung bei einer Temperatur von 0 bis 20 °C durchgeführt wird.